# EUROPEAN PATENT SPECIFICATION

(11) **EP 1 667 652 B1**
(45) Date of publication and mention of the grant of the patent: **30.05.2007**
(21) Application number: 04788968.8
(22) Date of filing: 24.09.2004
(51) Int. Cl.: A61K 9/00

(54) **IMPROVED CONTROLLED RELEASE DOSAGE FORM INCLUDING A BANDED ENGINE**
VERBESSERTE DOSIERFORM MIT KONTROLLIERTER FREISETZUNG MIT STEHANTRIEB
FORME POSOLOGIQUE AMELIOREE A LIBERATION CONTROLEE COMPRENANT UN MOTEUR ATTACHE

(30) Priority: 26.09.2003 US 506563 P
(43) Date of publication of application: 14.06.2006
(73) Proprietor: ALZA CORPORATION, Mountain View, CA 94039-7210 (US)
(72) Inventor: YUM, Si-Hong, Belmont, CA 94002 (US); POLLOCK-DOVE, Crystal, Mountain View, CA 94040 (US); DONG, Liang-Chang, Sunnyvale, CA 94086 (US); WONG, Patrick S. L., Burlingame, CA 94010 (US)
(74) Representative: Williams, Paul Edwin
(86) International application number: PCT/US2004/031301
(87) International publication number: WO 2005/030164

(56) References cited:
- WO-A-00/13674
- WO-A-03/000230
- WO-A-03/053400

## Description

### BACKGROUND

Field of the Invention: The present invention relates to dosage forms suitable for providing the controlled release of a variety of active agent formulations, including liquid active agent formulations. More specifically, the present invention is directed to a dosage form configured for the controlled release of an active agent formulation that includes a reservoir and an engine banded to the reservoir, wherein the engine is formulated or configured to expel the active agent formulation from within the reservoir after administration of the dosage form.

State of tho Art: Dosage forms providing controlled release of liquid active agent formulations are known in the art. For example, U.S. Patents 5,245,357, 6,174,547, 5,830,502, and 5,614,578, U.S. Patent Applications numbered 10/324,154, 10/324,239, 09/733,847, 08/075,084, 60/492,002, and 60/392,774, and International Publications numbered WO 95/34285 and WO 01/41742, disclose various different dosage form designs and active agent formulations suitable for providing dosage forms capable of delivering a liquid active agent formulation at controlled rate over a desired period of time. The benefits of controlled delivery of active agents are well recognized in the art, and dosage forms that achieve controlled delivery of liquid active agent formulations bring the benefits of controlled delivery to active agents that are not well suited to administration from conventional solid or tableted formulations.
WO 03053400 discloses an "osmotic engine-active formulation system" completely encapsulated and surrounded by a coating semipermeable membrane.

As can be appreciated by references cited herein, dosage forms providing controlled release of liquid active agent formulations may be osmotically driven and created using reservoirs formed with various different hard or soft capsule materials. In addition, where a controlled release liquid active agent dosage form is osmotically driven, the osmotic engine included in such a dosage form may be coated on the outside surface of the reservoir or the osmotic engine may be encapsulated by the reservoir. Even further, as is taught in U.S. Patent Applications numbered 60/492,002 and 60/392,774 ("the '002 Application" and "the '774 Application," respectively), the osmotic engine may be only partly enclosed by the reservoir. Controlled release liquid active agent dosage forms that include engines that are positioned within the reservoir but are only partly encapsulated by reservoir forming material are presently thought to be advantageous. In particular, dosage forms that include an engine that is only partly encapsulated by the reservoir are thought to exhibit improved structural stability and more effectively preserve release rate functionality over time, especially where the engine included in the dosage form is an osmotic engine.

Despite the benefits provided by controlled release dosage forms that include an engine only partly encapsulated by the reservoir, dosage forms designed according to the teachings of the '002 Application and the '774 Application present manufacturing challenges. For example, the engine included in such dosage forms is positioned within the reservoir prior to one or more coating steps required to finish the dosage form. However, because the engine is held in place through a friction fit, the engine may be displaced or separated from the reservoir as pressure is exerted against the reservoir or the reservoir and engine are subjected to other mechanical stresses during the manufacturing process. Separation or displacement of the engine may be particularly problematic at commercial production scales, as the product batches are typically subjected to various mechanical stresses during automated production processes and the batch sizes are relatively large, which can magnify the stresses exerted against each dosage form due to the number and collective weight of the dosage forms included in each batch. Moreover, because the liquid active agent formulation may be loaded within the reservoir before placement of the engine, separation of the engine from the reservoir during subsequent manufacturing steps is particularly undesirable, as it not only results in the manufacture of a defective dosage form, but can also lead to the loss of active agent and contamination of an entire process batch.

Even where the engine and reservoir of dosage forms designed according to the teachings of the '002 and '774 Applications do not separate during fabrication, the mechanical integrity of the finished dosage forms may be less than desired. In particular, where the opening formed in the reservoir and the engine interface, a step is produced on the outside surface of the dosage form, and as one or more coatings are provided over the reservoir and engine to secure the engine in place and provide a finished dosage form, the step formed on the outside surface may create point of discontinuity or reduced coverage in the coating materials. A point of discontinuity or reduced coating coverage may result in an area of weakness, and where an area of weakness exists, the application of pressure to the dosage form may cause cracking of the coatings, separation of the engine from the reservoir, or leaking of the liquid active agent formulation. To overcome this problem, the one or more outer coatings may be created under relatively wet coating conditions. However, to achieve the desired coating continuity, the coating conditions must typically be so wet that the tackiness of the coatings causes an undesirable increase in the rate at which dosage forms processed in the same batches adhere to each other, producing "twins" or groups of defective dosage forms.

It would be an improvement in the art, therefore, to provide a controlled release dosage form that is capable of delivering liquid active agent formulations, offers the benefits achieved by dosage forms such as those taught in the '002 and '774 Applications, and is better suited to commercial scale manufacture. Specifically, it would be an improvement in the art to provide a controlled release dosage form that is capable of delivering liquid active agent formulations, includes an engine only partially encapsulated by the reservoir containing the active agent formulation, and is designed to more effectively retain the engine at a proper position within the reservoir as the dosage form is manufactured. Ideally, the design of such a dosage form would also ease subsequent coating of the engine and reservoir, would not compromise release rate functionality and would allow the delivery of a wide range of liquid active agent formulations at various different controlled rates.

### SUMMARY OF THE INVENTION

In one aspect, the present invention is directed to a dosage form configured to provide the controlled release of an active agent formulation. A dosage form according to the present invention includes a reservoir containing an active agent formulation and an osmotic engine positioned at least partially within the reservoir. The opening of the reservoir and the engine included in a dosage form of the present invention are sized and shaped such that the engine can be received within the opening and positioned such that at least a portion of the engine extends into the reservoir. Moreover, the engine and the reservoir are configured such that, once the engine is positioned within the opening of the reservoir, the osmotic engine is not completely encapsulated by the reservoir. The dosage form of the present invention is designed and configured in a manner that provides a dosage form that operates to expel the active agent formulation from within the reservoir at a controlled rate after administration of the dosage form to an environment of operation.

In order to reduce the possibility that the engine included in a dosage form of the present invention will separate from the reservoir either during or after fabrication, the dosage form of the present invention includes a band that binds the engine to the reservoir. The band is provided over an outside surface of the both the engine and the reservoir at or near the interface formed where the engine enters the opening provided in the reservoir. Banding the engine of the dosage form of the present invention to the reservoir not only serves to reduce the frequency with which the engine separates from the reservoir, but also works to provide a smoother material transition where the outside surface of the engine meets the opening formed in the reservoir. Moreover, banding the engine to the reservoir can work to enhance the seal produced at the interface of the engine and the reservoir such that the likelihood that the active agent formulation leaking from the reservoir by passing around the engine is reduced.

In another aspect, the present invention is directed to a method of manufacturing a controlled release dosage form. In each embodiment, the method of the present invention includes providing a reservoir having an opening that is sized and shaped to receive an osmotic engine, providing an osmotic engine, positioning the engine within the opening of the reservoir and banding the engine to the reservoir. The step of banding the engine to the reservoir takes place after the engine is positioned within the opening of the reservoir. The method of the present invention also includes loading an active agent formulation into the reservoir, and configuring the dosage form of the present invention such that an exit orifice is included or formed in the reservoir to allow delivery of the active agent formulation. Though the active agent is preferably loaded before the engine is positioned within and banded to the reservoir, loading the active agent formulation in the dosage form of the present invention may also take place after the engine and reservoir have been operatively associated and banded.

### BRIEF DESCRIPTION OF THE DRAWINGS

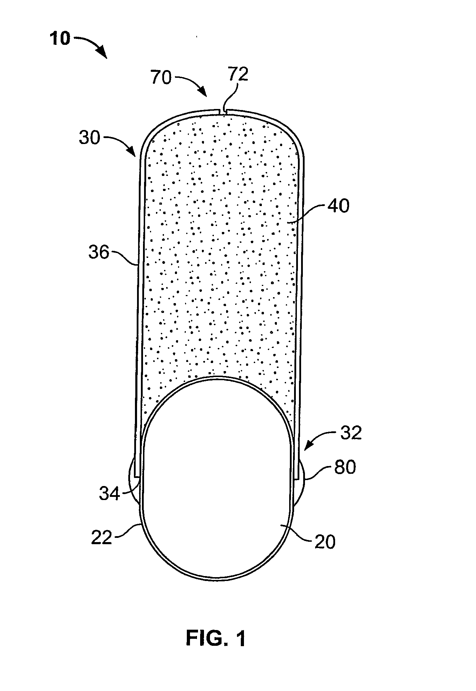

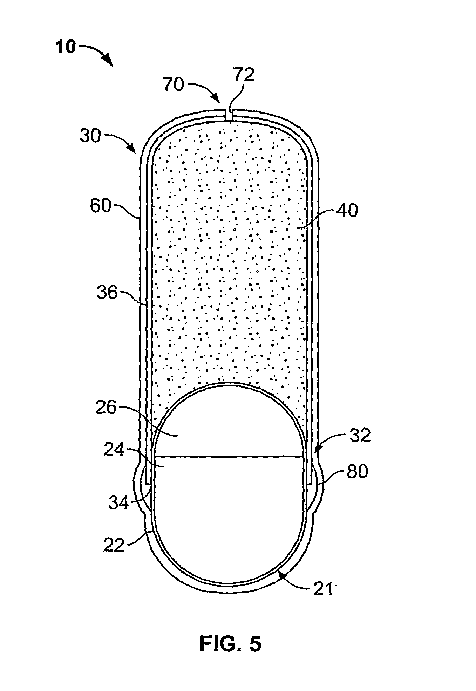

FIG. 1 through FIG. 6 provide cross-sectional representations of different embodiments of the dosage form of the present invention.

### DETAILED DESCRIPTION OF THE INVENTION

In one aspect, the present invention is directed to a dosage form. Various different embodiments of the dosage form 10 of the present invention are illustrated in FIG. 1 through FIG. 6. A dosage form 10 according to the present invention includes an engine 20 and a reservoir 30 suitable for containing an active agent formulation 40. The reservoir 30 and engine 20 are associated such that, as the dosage form 10 functions, the engine 20 operates to expel the active agent formulation 40 from within the reservoir 30 at a desired rate. In particular, the reservoir 30 of a dosage form of the present invention includes an opening 34, and the opening 34 of the reservoir 30 and engine 20 are sized and shaped to permit at least partial insertion of the engine 20 within the reservoir 30 through the opening 34.

The dosage form 10 of the present invention also includes a band 80 positioned at the step formed by the outside surface of the reservoir 36 and the outside surface 22 of the engine20 where the engine 20 enters the opening 34 formed in the reservoir 30. The material forming the band 80 extends around the dosage form 10, such that the band 80 is formed continuously around the dosage form 10 in the area where the engine 20 and reservoir 30 come together. The band 80 works to both bind the engine 20 and the reservoir 30 together and to reduce the step created on the outside surface of the dosage form where the engine 20 and reservoir 30 meet.

The dosage form 10 of the present invention may be provided with any desired active agent formulation 40 that can be delivered from the dosage form 10. As it used herein, the expression "active agent" encompasses any drug, therapeutic compound, or composition that can be delivered to provide a benefit to an intended subject or environment. The expression "active agent formulation" is used herein to indicate a formulation that contains an active agent and can be discharged from a dosage form of the present invention as the dosage form operates in a desired environment of use. An active agent formulation 40 suitable for use in the dosage form 10 of the present invention is preferably a liquid formulation and may be neat liquid active agent or a solution, suspension, slurry, emulsion, self emulsifying composition, liposomal composition, or other flowable formulation in which the active agent is present. The active agent formulation 40 may also be solid, or not flowable, before administration of the dosage form 10 to a desired environment of operation. However, where the active agent formulation 40 included in the dosage form 10 of the present invention is a solid formulation before administration, the formulation becomes flowable after administration. A solid active agent formulation may become flowable after administration due to, for example, the relatively higher temperature of the operational environment or the uptake of water into the active agent formulation.

A binder, antioxidant, pharmaceutically acceptable carrier, permeation enhancer, or the like may accompany the active agent in the active agent formulation 40. Further, the active agent formulation 40 may include a surfactant of mixture of surfactants. U.S. patents 6,174,547 and 6,245,357 and U.S. patent applications numbered 08/075,084, 09/733,847, 10/324,154, and 10/343,001, detail exemplary drugs, carriers, and other constituents that may be used to form a active agent formulation 40 suitable for use in the dosage form 10 of the present invention.

The reservoir 30 included in a dosage form 10 of the present invention is formed to contain a desired amount of active agent formulation 40 and may be formed as desired to accommodate the engine 20. For example, the reservoir 30 can be formed with a first end 32 that includes an opening 34 that is sized and shaped to accommodate an engine 20 that operates to drive the active agent formulation from within the reservoir 30. Moreover, though the reservoir 30 of a dosage form 10 of the present invention may be formed in a generally oblong shape, the dosage form 10 according to the present invention is not so limited and may be manufactured to include a reservoir 30 that is sized and shaped as desired to suit a particular dosage form or active agent delivery application.

Though it may be formed in various shapes and sizes and includes an opening 34 designed to receive an engine 20, the reservoir 30 included in a dosage form 10 of the present invention does not completely enclose or encapsulate the engine 20. As is described in U.S. Patent Application No. 60/492,002 and U.S. Patent Application No. 60/392,774, designing a controlled release active agent dosage form to include a reservoir 30 that does not completely encapsulate the engine 20 can result in a dosage form that is easier to manufacture, exhibits improved structural stability, and better preserves release rate functionality. Moreover, designing a controlled release active agent dosage form to include a reservoir 30 that does not entirely encapsulate the engine 20 can facilitate the use reservoirs formed of a wider range of materials. For example, where the engine 20 included in a dosage form 10 of the present invention is an osmotic engine 21, the proper function of the engine 20 depends on an influx of water from an environment of operation. If the reservoir 30 is formed of a water impermeable material and is configured such that the reservoir 30 completely encloses the engine 20, the engine 20 could not function as desired to provide the controlled release of an active agent formulation 40.

The reservoir 30 included in a dosage form 10 of the present invention may be formed of a variety of materials. Any material that is compatible with a desired active agent formulation, is capable of being formed into a reservoir of desired shape and size, is suitable for administration to a desired environment of operation, and is capable of withstanding the anticipated storage conditions and operational stresses can be used to provide the reservoir 30 included in a dosage form 10 according to the present invention. Depending on the active agent formulation 40 included in the dosage form 10 and the desired performance characteristics of the dosage form 10, the reservoir 30 may be formed of a water permeable material or a material that is impermeable to water. A reservoir 30 useful in a dosage form according to the present invention may be fabricated by any suitable method. Examples of materials and methods that may be used to form a reservoir to be used in a dosage form 10 of the present invention are described in, for example, U.S. Patents 6,183,466, 6,174,547, 6,153,678, 5,830,502, and 5,614,578, in U.S. Patent Applications numbered 10/324,154, 10/324,239, 09/733,847, 08/075,084, 60/492,002, and 60/392,774.

Water permeable materials that may be used to form a reservoir 30 included in a dosage form 10 of the present invention include, for example, materials typically used to fabricate orally deliverable, liquid filled capsules. A water permeable reservoir 30 included in a dosage form 10 of the present invention may be formed using hydrophilic polymer materials or hydrophilic gelatin materials. Hydrophilic polymer materials, including cellulosic materials, provide preferred water permeable materials that may be used to form a reservoir 30 useful in a dosage form 10 of the present invention. Relative to the gelatin materials that are typically used in dosage form fabrication, water-soluble polymer materials are less susceptible to moisture loss and are less sensitive to changes in moisture content. As a result, a reservoir 30 formed using a hydrophilic polymer material may be better able to retain its structural integrity upon exposure to the active agent formulation 40 and the engine 20 included in a dosage form 10 of the present invention, particularly where the engine 20 is an osmotic engine 21 that exerts a high osmotic pressure. Moreover, because hydrophilic polymer materials are generally less susceptible to moisture loss, a reservoir 30 manufactured using hydrophilic polymer materials can be made such that less water is available to be drawn into the active agent formulation 40 from within the materials forming the reservoir 30 itself. Therefore, where a reservoir 30 of a dosage form 10 of the present invention is formed using a water permeable material, it is presently preferred that the water permeable material be formed of a hydrophilic polymer material.

Hydrophilic polymer materials that may be used to as the water permeable material included in a multilayer reservoir 30 include, but are not limited to, polysaccharide materials, such as hydroxypropylmethyl cellulose (HPMC), methylcellulose, hydroxyethyl cellulose (HEC), hydroxypropyl cellulose (HPC), poly(vinylalcohol-co-ethylene glycol) and other water soluble polymers. Though the water permeable material included in a reservoir 30 of a dosage form 10 of the present invention may be manufactured using a single polymer material, the water permeable material may also be formed using a mixture of more than one polymer. Presently, because HPMC capsules for oral delivery of active agent formulations are commercially available and it has been found that capsule bodies formed of HPMC can be used to provide a reservoir 30 exhibiting suitable performance characteristics, the water permeable material included in a reservoir 30 of a dosage form 10 of the present invention is preferably formed using an HPMC material.

Where the reservoir 30 is formed of a material that is impermeable to water, the reservoir 30 can be made using a single material or a combination of materials. The material used to create a reservoir 30 that is suitable for use in a dosage form 10 according to the present invention and is impermeable to water according to the present invention need not be perfectly impermeable to the passage of water. As it is used herein, the term "impermeable" refers to reservoir formed of a material that exhibits a water flux of less than about 10⁻⁴ (mil·cm/atm·hr). Where the reservoir 30 included in a dosage form 10 of the present invention is formed using a water impermeable material, the water impermeable nature of the material serves to reduce or prevent migration of water from an external environment, through the reservoir 30, and into the active agent formulation 40.

In one embodiment, a water impermeable reservoir 30 suitable for use in a dosage form 10 according to the present invention is formed using a single layer of material that is impermeable to the passage of water. Materials suitable for forming such a reservoir 30 include, but are not limited to, water impermeable polymer materials. Where a single layer of water impermeable polymer material is used to form the reservoir 30, the polymer is preferably a synthetic resin or a combination of synthetic resins. Examples of water impermeable synthetic resins that may be used to form the reservoir 30 include, for example, linear polycondensation resins, condensation polymerized resins, addition polymerized resins, resins of phthalic anhydrides, polyvinyl resins such as polyethylene, polypropylene and their copolymers, polymer resins of methacrylic acid esters and acrylic acid esters, polycaprolactone, and copolymers of polycaprolactone with dilactide, diglycolide, valerolactone or decalactone. Different impermeable polymer materials and different combinations of impermeable polymer materials may be chosen to provide a reservoir 30 providing desired permeability, compatibility, and stability characteristics. A water impermeable reservoir may be formed, for example, using coating or molding techniques that are known in the art, such as, for example, those techniques described in U.S. Patents 6,183,466, 6,153,678, 5,830,502, and 5,614,578 and in U.S. Patent Applications numbered 60/492,002 and 60/392,774.

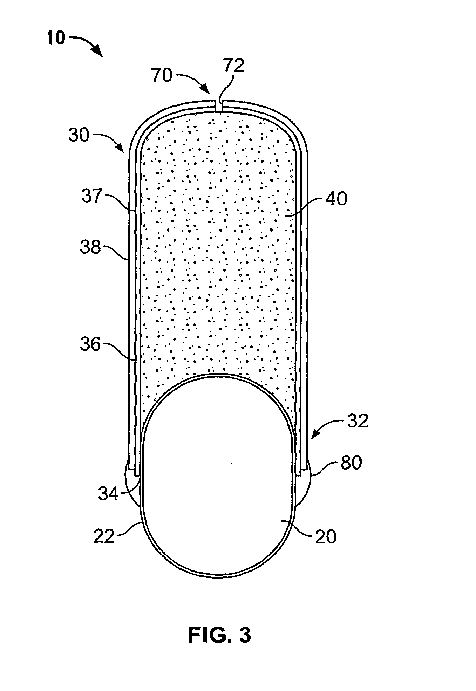

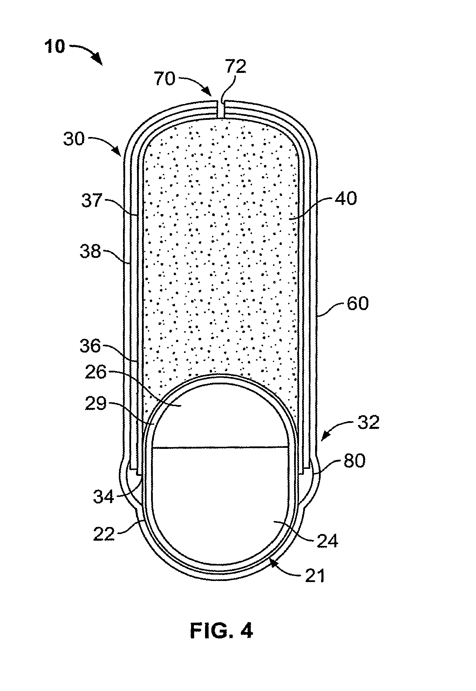

In an alternative embodiment, a water impermeable reservoir 30 included in a dosage form 10 according to the present invention may include two or more layers of different materials. For example, as is illustrated in FIG. 3 and FIG. 4, a reservoir 30 of a dosage form 10 of the present invention can include a water permeable material 37 coated with a water impermeable subcoat 38. The water permeable material 37 may be formed of a substance that is hydrophilic or otherwise permeable to the passage of water, such as the hydrophilic polymer and gelatin materials already described herein. The water permeable material 37 included in a water impermeable reservoir 30 included in a dosage form 10 according to the present invention may also be formed of a combination of water permeable and water impermeable materials. The water permeable material included in such a reservoir 30 may be formulated and formed by known methods, such as by the techniques described herein as useful in forming a water permeable reservoir 30 formed of a hydrophilic polymer or gelatin material. A water impermeable subcoat 38 included in a reservoir 30 of a dosage form 10 according to the present invention may be formed using any suitable water impermeable material that can be coated on or otherwise provided over the water permeable material 37. However, latex materials, such as Surelease® latex materials, which are available from Colorcon, Inc., Kollicoat® SR latex materials, which are available from BASF, Eudragit® SR, and other polymethylacrylate latex materials, are presently preferred for forming a water impermeable subcoat 38. A water impermeable subcoat 38 may be provided over the water permeable material 37 included in a water impermeable reservoir 30 of a dosage form according to the present invention using any suitable coating or lamination technique. Coating processes suitable for providing a water impermeable subcoat 38 are described, for example, in U.S. Patent Applications numbered 60/492,002, and 64/392,774.

The engine 20 included in a dosage form 10 of the present invention is an osmotic engine 21.
After administration of the dosage form to an environment of operation, the engine 10 included in a dosage form of the present invention operates by exerting a force against the active agent formulation 40 included in the reservoir 30 over a desired period of time, which force is sufficient to expel the active agent formulation 40 from within the reservoir 30.

In order to avoid any problems associated with permeation of the engine 20 by the active agent formulation 40 included in the dosage form 10, the engine 20 included in a dosage form 10 of the present invention is preferably resistant to permeation by the active agent formulation 40. As it is used herein, the terms "resistant to permeation" or "permeation resistant" refers to an engine that is configured or formulated such that, when included in a dosage form of the present invention, the engine exhibits an uptake of active agent formulation that is less than 5% by weight before administration of the dosage form. In preferred embodiments, the engine 20 included in the dosage form 10 of the present invention preferably exhibits an uptake of active agent formulation that is 3% by weight, or less, before administration of the dosage form, with engines exhibiting active agent formulation uptake of 1% by weight, or less, before administration of the dosage form being particularly preferred.

An osmotic engine 21 suitable for use in a dosage form 10 of the present invention includes an expandable osmotic composition 24 and is preferably prepared such that it is resistant to permeation by the active agent formulation 40 included in the dosage form.

An expandable osmotic composition 24 included in an osmotic engine 21 of a dosage form 10 according to the present invention may be formulated and formed using any materials and means that result in a composition that can be operatively associated with and bonded to the reservoir 30, is acceptable for the intended application of the dosage form 10, exhibits sufficient osmotic pressure to draw in water from an environment of operation over a desired period of time, and expands to exert a force sufficient to cause expulsion of an active agent formulation 40 from within a reservoir 30 as water is taken into the composition. The expandable osmotic composition 24 included in an osmotic engine 21 useful in a dosage form 10 of the present invention can be manufactured using known materials and methods, and may be formulated to provide an expandable osmotic composition 24 that is itself resistant to permeation by the active agent formulation 40 or can be made permeation resistant. Presently, the expandable osmotic composition 24 included in an osmotic engine 21 of a dosage form of the present invention is preferably formed as a tableted composition that includes a hydrophilic polymer capable of swelling or expanding upon interaction with water or aqueous biological fluids.

The expandable osmotic composition 24 included in an osmotic engine 21 used in a dosage form of the present invention may further include an osmotic agent, or "osmagent," to increase the osmotic pressure exerted by the expandable osmotic composition 24, a suspending agent to provide stability and homogeneity to the expandable osmotic composition 24, a tableting lubricant, an antioxidant, or a non-toxic colorant or dye. Materials and methods that can be used to form an expandable osmotic composition 24 suitable for use in an osmotic engine 21 useful in a dosage form 10 of the present invention are taught, for example, in U.S. Patents 6,174,547 and 6,245,357 and in U.S. Patent Applications numbered 10/324,154, 10/324,239, 09/733,847, 08/075,084, 60/492,002, and 60/394,774.

An osmotic engine 21 included in a dosage form of the present invention may also include a barrier layer 26. A barrier layer 26 included in an osmotic engine 21 used in a dosage form 10 according to the present invention is formulated of composition that is substantially impermeable to the active agent formulation 40. The barrier layer 26 works to reduce permeation of the expandable osmotic composition 24 by the active agent formulation 40. In addition, the barrier layer 26 serves to increase the uniformity with which the driving power of the expandable osmotic composition 24 is transferred to the active agent formulation 40. Where an osmotic engine 21 included in a dosage form 10 of the present invention includes a barrier layer 26, the barrier layer 26 and expandable osmotic composition 24 may be formed as a bi-layer tablet 28. Materials and methods suitable for creating such a bi-layer tablet 28 are taught, for example, in U.S. patent applications numbered 08/075,084, 60/343,001, and 60/343,005.
Materials suitable for forming a barrier layer 26 useful in an osmotic engine 21 used in a dosage form 10 according to the present invention include, but are not limited to, a polymeric composition, a high density polyethylene, a wax, a rubber, a styrene butadiene, a calcium phosphate, a polysilicone, a nylon, Teflon®, a polystyrene, a polytetrafluoroethylene, halogenated polymers, a blend of a microcrystalline, high acetyl cellulose, or a high molecular weight fluid impermeable polymer.

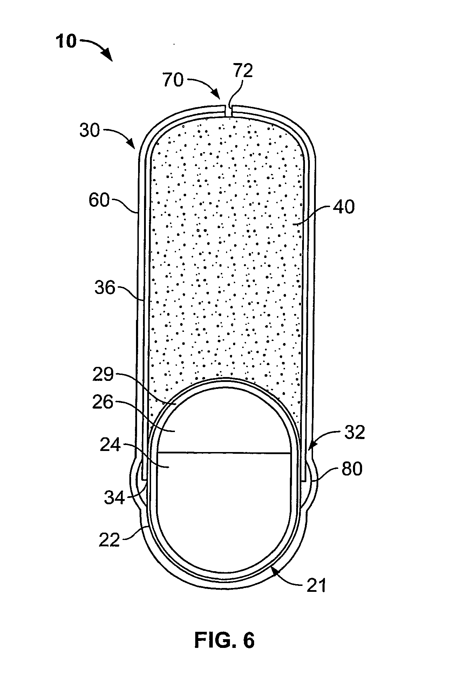

Where desired, an osmotic engine 21 included in a dosage form 10 of the present invention may be a permeation resistant engine. A permeation resistant osmotic engine 21 useful in a dosage form 10 of the present invention may include an expandable osmotic composition 24 that is formulated to be permeation resistant as defined herein. However, where the expandable osmotic composition 24 included in an osmotic engine 21 according to the present invention is formed of a tableted, hydrophilic polymer composition, the expandable osmotic composition 24 will typically require further processing in order to render the expandable osmotic composition resistant 24 to permeation by an active agent formulation 40. For example, as is shown in FIG. 4 and FIG. 6, the expandable osmotic composition 24 may be provided with a permeation resistant coating 29 over at least an area of the expandable osmotic composition 24, wherein the coating 29 is formulated to be resistant to permeation by a given active agent formulation 40.

The materials used to form a permeation resistant coating 29 included in a permeation resistant osmotic engine 21 useful in a dosage form 10 of the present invention will vary depending on the nature of the active agent formulation 40 to which the expandable osmotic composition 24 must be made permeation resistant. In particular, to render the expandable osmotic composition 24 resistant to permeation by a hydrophobic active agent formulation, a permeation resistant coating 29 provided over the expandable osmotic composition will typically be a hydrophilic coating that is substantially impermeable to the hydrophobic active agent formulation. Alternatively, to render the expandable osmotic composition 24 resistant to permeation by a hydrophilic active agent formulation, a permeation resistant coating 29 provided over the expandable osmotic composition will typically be a hydrophobic coating that is substantially impermeable to the hydrophilic active agent formulation. As used herein, "substantially impermeable" refers to a coating composition that is sufficiently impermeable to an active agent formulation to render the expandable osmotic composition permeation resistant as defined herein. A permeation resistant coating 29 may be formulated using a variety of different naturally derived or synthetic materials, with materials and methods suitable for provide an permeation resistant osmotic engine being detailed in U.S. Patent Application numbered 60/492,002.

Where desired, a permeation resistant coating 29 may be formulated using blends of materials that provide desirable coating characteristics. For example, in order to achieve a permeation resistant coating 29 having desirable coating characteristics, it may be necessary to formulate the coating material using blends of film forming materials. In addition, a permeation resistant coating 29 according to the present invention may include one materials, such as a plasticizer, that improve the coating characteristics provided by a film forming material or a blend of film forming materials. In particular, where HPMC is used to form a permeation resistant coating 29 included in a permeation resistant engine useful in a dosage form 10 of the present invention, it is presently preferred that the HPMC coating is formulated using a plasticizer, such as PEG 8000. Importantly, a permeation resistant coating 29 is preferably formulated such that tensile strength of the permeation resistant coating 29 can be overcome by the force exerted by the expandable osmotic composition 24 as the osmotic engine 21 functions and the expandable osmotic composition 24 expands.

Where an engine 20 included in a dosage form of the present invention includes a permeation resistant coating 29 that is permeable to the passage of water, such as a coating that includes a hydrophilic polymer or water soluble component, the permeation resistant coating 29 may completely encapsulate the material or mechanism forming the engine 24. A permeation resistant coating 29 that encapsulates the expandable osmotic composition 24 included in an osmotic engine 21 is formulated to exhibit a water permeability that is sufficient to permit water to enter the expandable osmotic composition 24 at a rate that allows the osmotic engine 21 to expand as needed to provide a desired release rate of active agent formulation 40. Moreover, if desired, where a permeation resistant coating 29 is provided over an osmotic engine 21, the thickness and water permeability of a permeation resistant coating 29 may be adjusted to provide a further measure of control over the release characteristics of the dosage form 10. For example, in order to delay delivery of an active agent formulation 40 from a dosage form that incorporates an osmotic engine 21 having a permeation resistant coating 29 that encapsulates an expandable osmotic composition 24 and is permeable to water, the thickness of permeation resistant coating 29 may be increased until a desired delay is achieved.

However, a permeation resistant coating 29 included over an engine 20 included in a dosage form of the present invention need not entirely encapsulate the engine 20. In fact, where a permeation resistant coating 29 is included over an osmotic engine 21 and the permeation resistant coating 29 is impermeable to water or is not sufficiently permeable to water to allow the osmotic engine 21 to function as desired, the permeation resistant coating 29 is configured such that the permeation resistant coating 29 does not entirely encapsulate the expandable osmotic composition 24 including in the osmotic engine 21 (not shown). In that manner, the water can be taken up by the expandable osmotic composition 21 at a rate that enables the osmotic engine 21 to function as desired.

An osmotic engine 21 included in a dosage form 10 of the present invention can be configured to include a barrier layer 26 and a permeation resistant coating 29. Moreover, where an osmotic engine 21 includes both a permeation resistant coating 29 and a barrier layer 26, the barrier layer 26 may be included within the permeation resistant coating 29 or on an outside surface of the permeation resistant coating 29. Materials and methods for fabricating an osmotic engine that includes both a barrier layer 26 and a permeation resistant coating 29 are described in U.S. patent application 60/492,002.

A band 80 included in a dosage form of the present invention is formed after the engine 20 is positioned within the opening 34 of the reservoir 30, and the banding step preferably takes places before other further processing, such as coating the dosage form with a rate controlling membrane, take place. The material forming a band 80 provided in a dosage form 10 of the present invention does not completely cover portion 27 of the engine 20 left exposed by the reservoir 30 or the reservoir itself. The band 80 is formed or positioned at the step formed by the outside surface of the reservoir 36 and the outside surface 22 of the engine20 where the engine 20 enters the opening 34 formed in the reservoir 30. The material forming the band 80 extends around the dosage form 10, such that band 80 is formed continuously around the dosage form 10 in the area where the engine 20 and reservoir 30 come together. The band 80 works to both bind the engine 20 and the reservoir 30 together and to reduce the step created on the outside surface of the dosage form where the engine 20 and reservoir 30 meet.

Methods and materials that may be used to band the reservoir 30 to the engine 20 in a dosage form 10 of the present invention are taught, for example, U.S. Patents 6,365,183, 6,316,028, 6,020,000, 5,667,804, and 5,534,263 In particular, a band 80 included a dosage form 10 according to the present invention can be applied using a variety of techniques that include, but are not limited to, printing, such as Gravure-type printing, extrusion coating, screen coating, brush coating, spraying, painting, the Capsealer process developed by TAIT Design & Machine Co., Manheim, PA, and the process commonly referred to as the Quali-Seal^{®} process developed by Shionogi Qualicaps of Indianapolis, IN. Such systems and techniques can be modified to provide a band 80 of insoluble material in a dosage form of the present invention, which unlike previously banded dosage forms, does not include a capsule formed of a body and a cap that fits over the body and is not formed of a compressed matrix formulation.

Though the material forming the band 80 included in a dosage form of the present invention is preferably insoluble in water, the band 80 may also be formed using a material that is water soluble. An insoluble material suitable for forming a band 80 included in a dosage form of the present invention 10 includes any material suitable for joining the engine and the reservoir, can be applied at the interface formed between the reservoir and the engine where the engine is positioned within the reservoir, and maintains its physical and chemical integrity after administration of the dosage form, at least during the desired dispensing period of the dosage form. Preferably, an insoluble material used for form a band 80 included in the dosage form of the present invention is also biologically inert, nonallergenic and nonirritating to body tissue.

Specific insoluble materials that may be used to band the engine 20 to the reservoir 30 of a dosage form 10 of the present invention include, but are not limited to, polyethylene, polystyrene, ethylene-vinyl acetate copolymers, polycaprolactone and polyester based elastomers such as polyester/polyether block copolymers, including the HYTREL^{®} series of polymers available from DuPont. Additional insoluble banding materials include but are not limited to polysaccharides, cellulosics, powdered cellulose, microcrystalline cellulose, cellulose acetate, cellulose acetate pseudolatex (such as described in U.S. Pat. No. 5,024,842), cellulose acetate propionate, cellulose acetate butyrate, ethyl cellulose, ethyl cellulose pseudolatex (such as Surelease^{®}, as supplied by Colorcon, West Point, Pa. or Aquacoat^{TM} as supplied by FMC Corporation, Philadelphia, Pa.), nitrocellulose, polylactic acid, poly- glycolic acid, polylactide glycolide copolymers, collagen, polycaprolactone, polyvinyl alcohol, polyvinyl acetate, polyethylene vinylacetate, polyethylene teraphthalate, polybutadiene styrene, polyisobutylene, polyisobutylene isoprene copolymer, polyvinyl chloride, polyvinylidene chloride-vinyl chloride copolymer, copolymers of acrylic acid and methacrylic acid esters, copolymers of methylmethacrylate and ethylacrylate, latex of acrylate esters (such as Eudragit^{®} supplied by RohmPharma, Darmstaat, Germany), polypropylene, copolymers of propylene oxide and ethylene oxide, propylene oxide ethylene oxide block copolymers, ethylenevinyl alcohol copolymer, polysulfone, ethylene vinylalcohol copolymer, polyxylylenes, polyamides, natural and synthetic waxes, paraffin, carnauba wax, petroleum wax, white or yellow bees wax, castor wax, candelilla wax, rice bran wax, microcrystalline wax, stearyl alcohol, cetyl alcohol, bleached shellac, esterified shellac, chitin, chitosan, silicas, polyalkoxysilanes, polydimethyl siloxane, polyethylene glycol-silicone elastomers, crosslinked gelatin, zein, electromagnetic irradiation crosslinked acrylics, silicones, or polyesters, thermally crosslinked acrylics, silicones, or polyesters, butadiene-styrene rubber, glycerol ester of partially dimerized rosin, glycerol ester of partially hydrogenated wood rosin, glycerol ester of tall oil rosin, glycerol ester of wood rosin, pentaerythritol ester of partially hydrogenated wood rosin, pentaerythritol ester of wood rosin, natural or synthetic terpene resin and blends of the above.

Preferred insoluble banding materials include copolymers of acrylic acid and methacrylic acid esters, copolymers of methylmethacrylate and ethylacrylate, and latex of acrylate esters. Preferred copolymers include poly (butyl methacrylate, (2-dimethylaminoethyl)methacrylate, methyl methacrylate) 1:2:1, 150,000, sold under the trademark EUDRAGIT E; poly (ethyl acrylate, methyl methacrylate) 2:1, 800,000, sold under the trademark EUDRAGIT NE 30 D; poly (methacrylic acid, methyl methacrylate) 1:1, 135,000, sold under the trademark EUDRAGIT L; poly (methacrylic acid, ethyl acrylate) 1:1, 250,000, sold under the trademark EUDRAGIT L; poly (methacrylic acid, methyl methacrylate) 1:2, 135,000, sold under the trademark EUDRAGIT S; poly (ethyl acrylate, methyl methacrylate, trimethylammonioethyl methacrylate chloride) 1:2:0.2, 150,000, sold under the trademark EUDRAGIT RL; poly (ethyl acrylate, methyl methacrylate, trimethylammonioethyl methacrylate chloride) 1:2:0.1, 150,000, sold as EUDRAGIT RS. In each case, the ratio x:y:z indicates the molar proportions of the monomer units and the last number is the number average molecular weight of the polymer. An ethylacrylate methylmethylacrylate 2:1 copolymer latex is especially preferred.

Water soluble materials may also be used to band the reservoir 30 to the engine 20 included in a dosage form 10 of the present invention. Any water soluble material that is suitable for joining the engine 20 and the reservoir 30, can be applied at the interface formed between the reservoir 30 and the engine 20 where the engine 20 is positioned within the reservoir 30, and at least maintains its physical and chemical integrity prior to administration of the dosage form 10 may be used to form a band 80 useful in a dosage form 10 of the present invention. As is true of water insoluble materials for banding the reservoir 30 to the engine 20, a water soluble material used to form a band 80 in a dosage form 10 of the present invention is preferably biologically inert, nonallergenic and nonirritating to body tissue.

In addition to the coating techniques already described herein, a band 80 included in a dosage form of the present invention may be formed using a tape or preformed band of banding material positioned around the dosage form 10 in a manner that binds the engine 20 to the reservoir 30. Where the band 80 is formed using a tape or pre-formed band, the thickness of the tape or preformed band is chosen such that any step formed at the transition formed at the edges of the tape or pre-formed band is smaller or less severe than the step formed at the opening of the reservoir 34, where the reservoir 30 and the engine 20 interface. In particular, where the band 80 included in a dosage form of the present invention is formed using tape or a preformed band, the tape or preformed band will have a thickness that is less than the thickness of the reservoir 30 where the reservoir 30 and engine 20 interface. In preferred embodiments, a tape or preformed band used to form the band 80 of the dosage form 10 of the present invention will have a thickness that is less than 50% of the thickness of the reservoir 30 where the reservoir 30 and engine 20 interface, and in particularly preferred embodiments, a tape or preformed band used to form the band 80 of the dosage form 10 of the present invention will have a thickness that is less than 25% of the thickness of the reservoir 30 where the reservoir 30 and engine 20 interface. Moreover, the edges of a tape or preformed band used to form the band 80 included in the dosage form 10 of the present invention are preferably tapered such that the thickness of the tape or preformed band at the outside edges is less than the thickness in the center of the tape or preformed band. Such a configuration further reduces any material transition formed between the edges of the tape or preformed band and the outside surface of the reservoir 30 and engine 20.

Where a tape is used, the tape may or may not include an adhesive. If the tape does not include an adhesive, the tape may be adhered to the reservoir 30 and engine 20 using a suitable solvent or adhesive. Alternatively, a tape used to form the band 80 included in the dosage form 10 of the present invention may be formed of a shape memory or heat shrinking material, such as a shape memory or heat shrinking polymer material, which is processed during or after application such that a band 80 that maintains the engine 20 in place relative to the reservoir 30 is formed.

Where the band 80 included in the dosage form 10 of the present invention is provided by a preformed band of material, the preformed band is preferably initially sized such that the inside diameter of the preformed band is at least slightly lager than the outside diameter of the reservoir 30 where at the opening 34 where the reservoir 30 and engine 20 interface. IN one embodiment, the inner diameter of a preformed band used to form the band 80 of the dosage form 10 of the present invention is sized such that it can be positioned over the interface formed between the reservoir 30 and engine 20 and at least initially maintained in place by a friction or interference fit. A preformed band may be adhered more permanently to the dosage form 10 at the interface formed between the reservoir 30 and engine 20 using any suitable adhesive material. Alternatively, a preformed band may be adhered to the dosage form using a solvent that partially solubilizes the material forming the preformed band or a material included on the outside surface of the engine 20 or the reservoir 30 such that band is adhered to or fused to the dosage form 10 as the solvent is removed or evaporates. In a preferred embodiment, a preformed band used to form the band 80 included in the dosage form of the present invention is fabricated using a shape memory or heat shrinkable polymer. Such materials are known in the art and are commercially available. After positioning a preformed band made of a shape memory or heat shrinkable polymer over the interface formed between the reservoir 30 and engine 20, the preformed band is subjected to conditions (*e.g*., heat) that cause the band to shrink around the reservoir 30 and engine 20, thereby banding the engine 20 to the reservoir.

Regardless of the particular materials or methods used to create the band 80 included in the dosage form 10 of the present invention, banding the engine 20 to the reservoir 30 reduces the likelihood that the engine 20 will be displaced from a desired position or separated from the reservoir 30 as the dosage form is manufactured steps.
Moreover, banding the engine of the dosage form of the present invention to the reservoir works to smooth any discontinuity or step formed where the outside surface of the engine interfaces the opening formed in the reservoir, and by smoothing the interface between the engine and reservoir, the design of the dosage form of the present invention production of subsequent coatings that exhibit better continuity and are more robust using coating conditions that are less likely to result in loss of product due to "twinning" of dosage forms in process. Even further, the band 80 included in a dosage form 10 of the present invention works to more effectively seal the interface between the engine 20 and the reservoir 30 from penetration or passage by the active agent formulation 40. Therefore, banding the engine 20 to the reservoir 30 not only provides a physically more robust controlled release active agent dosage form that is better suited to commercial production, but can also provide a dosage form that is less susceptible to the undesirable loss or leaking of active agent formulation from within the reservoir.

The; dosage form 10 preferably includes a rate controlling membrane 60. A rate controlling membrane 60 included on a dosage form 10 of the present invention allows water or aqueous fluid from the desired environment of operation to enter the osmotic engine 21 at a controlled rate and thereby facilitates controlled expansion of the osmotic engine 21 and controlled delivery of the active agent formulation 40 from the dosage form 10. A rate controlling membrane 60 included in a dosage form 10 according to the present invention is non-toxic in the intended environment of operation and maintains its physical and chemical integrity during the operation of the dosage form 10, Adjusting the thickness or chemical make-up of the rate controlling membrane 60 can control the rate at which the expandable osmotic composition 24 included in an osmotic engine 21 expands after the dosage form 10 is administered. Therefore, a rate controlling membrane 60 included in a dosage form 10 of the present invention serves to control the release rate or release rate profile achieved by a dosage form 10.

A rate controlling membrane 60 for use in a dosage form 10 of the present invention may be formed using any material that is permeable to water, is substantially impermeable to the active agent, is pharmaceutically acceptable, and is compatible with the other components of the dosage form 10 of the present invention. Generally, a rate controlling membrane 60 will be formed as a semipermeable membrane using materials that include semipermeable polymers, semipermeable homopolymers, semipermeable copolymers, and semipermeable terpolymers. Semipermeable polymers are known in the art, as evidenced by the patent references cited herein and by U.S. Patent No. 4,077,407. In addition, semipermeable polymers can be made by processes known in the art, such as the procedures described in *Encyclopealia of Polymer Science and Technology,* Vol. 3, pages 325 to 354, 1964, published by Interscience Publishers, Inc., New York. A rate controlling membrane 60 included in the dosage form 10 of the present invention may also include a plasticizer to impart flexibility and elongation properties to the rate controlling membrane 60 or a flux regulating agent, such as a flux enhancing or a flux reducing agent, to assist in regulating the fluid permeability or flux through the rate controlling membrane 60.

A rate controlling membrane 60 included in a dosage form 10 according to the present invention is provided over at least the portion 27 of an osmotic engine 21 that is not enclosed or encapsulated by the reservoir 30. If desired, a rate controlling membrane 60 included in a dosage form 10 of the present invention may also be provided over both the reservoir 30 and the exposed portion 27 of the osmotic engine 21. Moreover, where a dosage form 10 according to the present invention includes a reservoir 30 that is permeable to water, a rate controlling membrane 60 included in the dosage form 10 preferably extends over both the reservoir 60 and the exposed portion 27 of the osmotic engine 21.

Methods for providing a rate controlling membrane 60 suitable for use in a dosage form 10 according to the present invention are known in the art and include any suitable coating technique, such as a suitable dip coating or spray coating process. Additional references describing materials and methods for fabricating rate controlling membranes suitable for use in a oral dosage form 10 of the present invention include, for example, U.S. patents 6,174,547 and 6,245,357 and U.S. patent applications numbered 10/324,154, 10/324,239, 09/733,847, 08/075,084, 60/492,002, and 60/392,774.

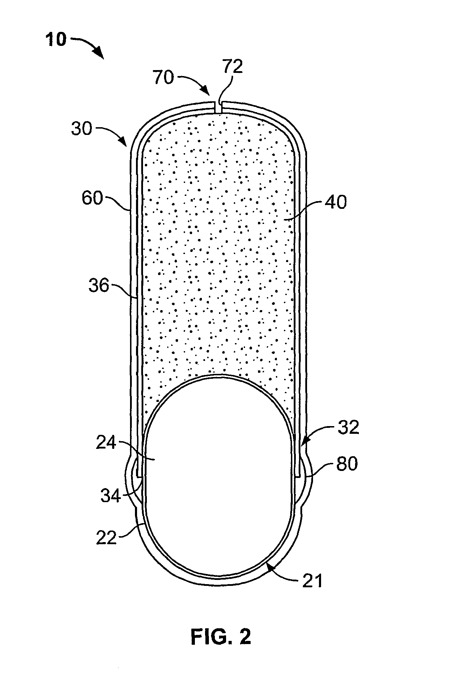

A dosage form 10 according to the present invention also includes an exit orifice 70. The exit orifice 70 may include any structure, device, or dosage form configuration that allows the active agent formulation 40 to be delivered from the reservoir 30 of the dosage form. An exit orifice 70 included in a dosage form 10 of the present invention may be embodied by one of various different structures. For example, the exit orifice 70 may include an aperture 72 formed partially or completely through the wall of the reservoir 30 included in the dosage form 10. Alternatively, as is shown in FIG. 2 and FIG. 4 through FIG. 6, where the dosage form 10 of the present invention includes a rate controlling membrane 60 over the reservoir 30, the exit orifice 70 may include an aperture 72 formed through the rate controlling membrane 60, or the exit orifice may include an aperture 72 formed through a rate controlling membrane 60 and a portion of the reservoir, such as a water impermeable subcoat 58 included in a reservoir 30 formed of multiple material layers. An exit orifice 70 formed of an aperture 72 may be formed by any suitable means, such as by suitable mechanical or laser drilling technologies.

Though the aperture 72 illustrated in FIG. 1 through FIG. 6 does not pass entirely through the reservoir 30 included in the dosage form 10, the aperture 72 allows the formation of an exit orifice as the dosage form is placed within or begins to operate within an intended environment of operation. In particular, where a dosage form 10 of the present invention includes a reservoir 30 formed of a single layer of water impermeable material, the aperture 72 formed in the rate controlling membrane 60 creates a breaking point where the material forming the reservoir 30 is compromised as the engine 20 included in the dosage form 10 begins to function and pressure within the reservoir 30 builds. Alternatively, where a dosage form 10 of the present invention includes a water permeable material and the aperture 72 exposes such material to the environment of operation, the water present in the environment of operation can work to weaken or dissolve the exposed portion of the reservoir 30, allowing the active agent formulation 40 contained within the reservoir 30 to be expelled as the engine 20 operates.

Nevertheless, the dosage form 10 of the present invention is not limited to an exit orifice 70 formed by an aperture 72. Where desired, the exit orifice may include an aperture that passes completely through the reservoir. Again, mechanical or laser drilling technologies may be used to create such an exit orifice. However, where the exit orifice provided in the dosage form of the present invention is formed through the reservoir, a closure sealing the exit orifice be needed. Any one of several means may be employed to provide such a closure. For instance, the closure may include a layer of material that covers the exit orifice and is arranged over a portion the outer surface of the dosage form, or the closure may include a stopper, such as a bung, cork, or impermeable plug, or an erodible element, such as a gelatin plug or a pressed glucose plug, formed or positioned within the exit orifice. Regardless of its specific form, the closure will typically comprise a material impermeable to the passage of the active agent formulation, at least until after administration of the dosage form. Suitable closure materials include high-density polyolefin, aluminized polyethylene, rubber, silicon, nylon, synthetic fluorine Teflon^{®}, chlorinated hydrocarbon polyolefins, and fluorinated vinyl polymers.

An exit orifice included in a dosage form of the present invention may also include more than a simple aperture, where desired, the exit orifice may include, for example, a porous element, porous overlay, porous insert, hollow fiber, capillary tube, microporous insert, or microporous overlay. Moreover, regardless of the particular structure providing the exit orifice, a dosage form of the present invention can be manufactured with two or more exit orifices for delivering the active agent formulation during operation. Descriptions of exit orifices suitable for use in controlled release dosage forms are disclosed, for example, in those patents and patent applications referred to herein as well as in U.S. patents numbered 3,845,770, 3,916,899, and 4,200,098.

Though an exit orifice 70 formed of an aperture 72 is only one of various different exit orifices that may be provided in a dosage form 10 of the present invention, exit orifices that are formed as shown in the illustrated embodiments are desirable, as they do not require complete penetration of the reservoir 30 before the dosage form 10 is administered. Such a design works to reduce the possibility that the active agent formulation 40 may leak from the dosage form 10 before the dosage form 10 is administered. Moreover, the aperture 72 included in the exit orifices 70 shown in FIG. 1 through FIG. 6 is simply formed using known mechanical or laser drilling techniques.

In another aspect, the present invention is directed to a method of manufacturing a dosage form providing the controlled release of a active agent formulation. The method of the present invention includes providing a reservoir including an opening, providing an osmotic engine, positioning the engine within the opening of the reservoir and banding the engine to the reservoir. The method of the present invention also includes loading an active agent formulation into the reservoir, and configuring the dosage form such that an exit orifice is included or formed in the reservoir to allow delivery of the active agent formulation. Though active agent is preferably loaded before the engine is positioned within and banded to the reservoir, loading the active agent formulation in the dosage form of the present invention may also take place after the engine and reservoir have been operatively associated.

The step of providing a reservoir including an opening may include providing any reservoir suitable for use in a dosage form of the present invention. For example, the reservoir provided in a method of the present invention may be formed of a water permeable or a water impermeable material, such as those materials disclosed herein. Moreover, the reservoir provided in a method of the present invention may be formed of a single layer of material or multiple layers of one or more different materials. The precise nature of the reservoir provided in a method according to the present invention will depend on, among other factors, the desired application and performance characteristics of the dosage form produced, as well as the nature of the engine and the active agent formulation to be included in the dosage form.

The engine provided in the method of the present invention is an osmotic engine and may include a barrier layer and may be formulated or configured to be resistant to permeation by the active agent formulation loaded in the reservoir. Where the engine provided in a method of the present invention is an osmotic engine that includes a barrier layer, the method of the present invention includes orienting the engine before the engine is positioned within the reservoir such that the barrier layer faces the active agent formulation in the completed dosage form. The precise nature of the engine provided in a method according to the present invention will depend on, among other factors, the desired application and performance characteristics of the dosage form produced, as well as the nature of the reservoir and the active agent formulation to be included in the dosage form.

The step of positioning the engine within the opening included in the reservoir can be carried out using any technique, device or mechanism that results in the desired positioning of the engine within the opening of the reservoir. For example, the positioning step may be carried out by an inserter providing insertion depth control or insertion force control. Preferably, an inserter providing insertion depth control is used to position the engine within the reservoir that has not already been loaded with an active agent formulation, while an inserter providing insertion force control is preferably used to position an engine within a reservoir that has been pre-loaded with an active agent formulation.

Loading the active agent formulation into the reservoir can also be carrier out by any technique, device or mechanism that results in the loading of a desired amount of active agent formulation in the reservoir. Where loading of the active agent formulation takes place before the engine is positioned within the opening of the reservoir, the active agent formulation may be loaded through the same opening used for positioning the engine. However, where the active agent formulation is loaded into the reservoir after positioning the osmotic engine, loading of the active agent formulation must be done either through a second opening formed in the reservoir or by passing the active agent formulation around the engine and into the reservoir. The active agent formulation loaded into the reservoir in a method according to the present invention may be any active agent formulation suitable for use in a dosage form according to the present invention.

The step of configuring the dosage form such that an exit orifice is included or formed in the reservoir may include forming one or more exit orifices as already described herein. For example, the method of the present invention may include creating one or more exit orifices that include a porous element, a porous overlay, a porous insert, a hollow fiber, a capillary tube, microporous insert, or microporous overlay, an aperture or an aperture with a closure, such as a layer of material positioned over the closure, an impermeable bung, cork, or plug, an erodible element, such as a gelatin plug or pressed glucose plug, formed or positioned within the aperature. Moreover, regardless of the particular structure providing the exit orifice, configuring the dosage form such that an exit orifice is included or formed in the reservoir may involve forming two or more exit orifices for delivering the active agent formulation during operation.

The engine provided in the method of the present invention is an osmotic engine, and the method of the present invention may also include providing a rate controlling membrane. Typically, the step of providing a rate controlling membrane includes providing a rate controlling membrane over at least the portion of the osmotic engine that is not encapsulated by the reservoir. Alternatively, depending on the type of material used to form the reservoir, the step of providing a rate controlling membrane may also include providing a rate controlling membrane over both the exposed portion of the osmotic engine and the reservoir. Where required, providing a rate controlling membrane can be carried out using any materials or methods suitable for creating a rate controlling useful in a dosage form according to the present invention. Particular examples of material and methods for providing a rate controlling membrane include, but are not limited to, those materials and methods described in U.S. Patents 6,174,547, 6,245,357 and 4,077,407, in U.S. Patent Applications numbered 10/324,154, 10/324,239, 09/733,847, 08/075,084, 60/492,002, and 60/392,774, and in *Encyclopedia of Polymer Science and Technology,* Vol. 3, pages 325 to 354, 1964, published by Interscience Publishers, Inc., New York.

Banding the engine to the reservoir of the dosage form in the method of the present invention can be carried out after the engine is positioned within the opening included in the reservoir and can be accomplished using, for example, the materials and methods discussed herein. For example, in one embodiment, the method of the present invention includes forming a band of water insoluble material on the outside surface of the reservoir and engine where the opening of the reservoir and the engine meet using a process selected from printing, such as Gravure-type printing, extrusion coating, screen coating, brush coating, spraying, painting, the Capsealer process developed by TAIT Design & Machine Co., Manheim, PA, and the process commonly referred to as the Quali-Seal^{®} process developed by Shionogi Qualicaps of Indianapolis, IN, and the like. In another embodiment, the method of the present invention includes forming a band of water soluble material on the outside surface of the reservoir and engine where the opening of the reservoir and.the engine meet using a process selected from those already described. In further embodiments, the method of the present invention includes banding the engine to the reservoir of the dosage form using a tape or preformed band of material. Materials and methods suitable for banding the engine to the reservoir using a tape or preformed band of material are described previously herein in relation to the formation of the dosage form of the present invention.

## Claims

1. A dosage form configured to provide the controlled release of an active agent formulation comprising
a reservoir containing an active agent formulation, an osmotic engine partially positioned within the reservoir, and the engine not being completely encapsulated by the reservoir, and
a band provided over an outer surface of the reservoir and the engine that binds the engine to the reservoir;
wherein the dosage form is configured to expel the active agent formulation from within the reservoir at a controlled rate after administration of the dosage form to an environment of operation.

2. The dosage form of claim 1, wherein the band is a printed band, extrusion coated band, screen coated band, brush coated band, sprayed band, or painted band.

3. The dosage form of claim 1, wherein the band comprises a band formed continuously around the dosage form in an area where the engine and reservoir come together.

4. The dosage form of claim 1, wherein the band comprises material selected from the group consisting of polyethylene, polystyrene, ethylene-vinyl acetate copolymers, polycaprolactone and polyester based elastomers, polysaccharides, cellulosics, powdered cellulose, microcrystalline cellulose, cellulose acetate, cellulose acetate pseudolatex, cellulose acetate propionate, cellulose acetate butyrate, ethyl cellulose, ethyl cellulose pseudolatex, nitrocellulose, polylactic acid, poly- glycolic acid, polylactide glycolide copolymers, collagen, polycaprolactone, polyvinyl alcohol, polyvinyl acetate, polyethylene vinylacetate, polyethylene teraphthalate, polybutadiene styrene, polyisobutylene, polyisobutylene isoprene copolymer, polyvinyl chloride, polyvinylidene chloride-vinyl chloride copolymer, copolymers of acrylic acid and methacrylic acid esters, copolymers of methylmethacrylate and ethylacrylate, latex of acrylate esters, polypropylene, copolymers of propylene oxide and ethylene oxide, propylene oxide ethylene oxide block copolymers, ethylenevinyl alcohol copolymer, polysulfone, ethylene vinylalcohol copolymer, polyxylylenes, polyamides, natural and synthetic waxes, paraffin, carnauba wax, petroleum wax, white or yellow bees wax, castor wax, candelilla wax, rice bran wax, microcrystalline wax, stearyl alcohol, cetyl alcohol, bleached shellac, esterified shellac, chitin, chitosan, silicas, polyalkoxysilanes, polydimethyl siloxane, polyethylene glycol-silicone elastomers, crosslinked gelatin, zein, electromagnetic irradiation crosslinked acrylics, silicones, or polyesters, thermally crosslinked acrylics, silicones, or polyesters, butadiene-styrene rubber, glycerol ester of partially dimerized rosin, glycerol ester of partially hydrogenated wood rosin, glycerol ester of tall oil rosin, glycerol ester of wood rosin, pentaerythritol ester of partially hydrogenated wood rosin, pentaerythritol ester of wood rosin, natural or synthetic terpene resin and blends of the above.

5. The dosage form of claim 1, wherein the band comprises a tape.

6. The dosage form of claim 1, wherein the band comprises a preformed band.

7. The dosage form of claim 1 , wherein the osmotic engine comprises an expandable osmotic composition.

8. The dosage form of claim 1, wherein the osmotic engine comprises a barrier layer or an outer coating that limits migration of an active agent formulation from the reservoir into the osmotic engine.

9. The dosage form of claim 1, wherein the reservoir comprises a water permeable material.

10. The dosage form of claim 1, wherein the reservoir comprises a material that is substantially impermeable to water.

11. A dosage form comprising
a reservoir containing an active agent formulation,
an osmotic engine partially positioned within an opening formed within the reservoir, and the osmotic engine not being completely encapsulated by the reservoir,
a band provided over an outer surface of the reservoir and the engine that binds the engine to the reservoir,
a rate controlling membrane, and an exit orifice through which the active agent formulation can be delivered.

12. A method of manufacturing a dosage form providing the controlled release of an active agent formulation comprising:
providing a reservoir having an opening that is sized and shaped to receive an osmotic engine,
providing an osmotic engine
positioning the engine within the opening of the reservoir so that the engine partially is positioned within the reservoir, and the engine not being completely encapsulated by the reservoir, and
banding the engine that to the reservoir.

13. The method of claim 12, wherein banding the engine to the reservoir comprises printing the band, extrusion coating the band, screen coating the band, brush coating the band, spraying the band, or painting the band.

14. The method of claim 12, wherein banding the engine to the reservoir comprises a forming the band continuously around the dosage form in an area where the engine and reservoir come together.

15. The method of claim 12, wherein the band comprises material selected from the group consisting of polyethylene, polystyrene, ethylene-vinyl acetate copolymers, polycaprolactone and polyester based elastomers, polysaccharides, cellulosics, powdered cellulose, microcrystalline cellulose, cellulose acetate, cellulose acetate pseudolatex, cellulose acetate propionate, cellulose acetate butyrate, ethyl cellulose, ethyl cellulose pseudolatex, nitrocellulose, polylactic acid, poly- glycolic acid, polylactide glycolide copolymers, collagen, polycaprolactone, polyvinyl alcohol, polyvinyl acetate, polyethylene vinylacetate, polyethylene teraphthalate, polybutadiene styrene, polyisobutylene, polyisobutylene isoprene copolymer, polyvinyl chloride, polyvinylidene chloride-vinyl chloride copolymer, copolymers of acrylic acid and methacrylic, acid esters, copolymers of methylmethacrylate and ethylacrylate, latex of acrylate esters, polypropylene, copolymers of propylene oxide and ethylene oxide, propylene oxide ethylene oxide block copolymers, ethylenevinyl alcohol copolymer, polysulfone, ethylene vinylalcohol copolymer, polyxylylenes, polyamides, natural and synthetic waxes, paraffin, carnauba wax, petroleum wax, white or yellow bees wax, castor wax, candelilla wax, rice bran wax, microcrystalline wax, stearyl alcohol, cetyl alcohol, bleached shellac, esterified shellac, chitin, chitosan, silicas, polyalkoxysilanes, polydimethyl siloxane, polyethylene glycol-silicone elastomers, crosslinked gelatin, zein, electromagnetic irradiation crosslinked acrylics, silicones, or polyesters, thermally crosslinked acrylics, silicones, or polyesters, butadiene-styrene rubber, glycerol ester of partially dimerized rosin, glycerol ester of partially hydrogenated wood rosin, glycerol ester of tall oil rosin, glycerol ester of wood rosin, pentaerythritol ester of partially hydrogenated wood rosin, pentaerythritol ester of wood rosin, natural or synthetic terpene resin and blends of the above..

16. The method of claim 12, wherein the band comprises a tape.

17. The method of claim 12, wherein the band comprises a preformed band.

18. The method of claim 12, further comprising loading an active agent formulation into the reservoir

19. The method of claim 12, further comprising
configuring the dosage form such that an exit orifice is included or formed in the reservoir to allow delivery of the active agent formulation.

20. The method of claim 12, wherein providing an engine comprises providing an osmotic engine that comprises a rate controlling membrane

21. The method of claim 20 wherein the rate controlling membrane is formed or positioned over at least a portion of the osmotic engine that is not encapsulated by the reservoir.

22. The method of claim 20, wherein the controlling membrane is formed or positioned over both an exposed portion of the osmotic engine and the reservoir.

23. The method of claim20, wherein the osmotic engine further comprises a barrier layer

24. The method of claim 23, further comprising:
orienting the osmotic engine before it is positioned within the reservoir such that after the engine is positioned within the opening of the reservoir, the barrier layer faces the active agent formulation.

25. The method of claim 23, wherein the barrier layer comprises a barrier layer that is resistant to permeation by the active agent formulation.

## Patentansprüche

1. Darreichungsform, die gestaltet ist, um die kontrollierte Freisetzung einer Formulierung mit aktivem Mittel bereitzustellen, umfassend:
ein Reservoir, das die Formulierung mit aktivem Mittel enthält, einen osmotischen Antrieb, der teilweise innerhalb des Reservoirs positioniert ist und der Antrieb nicht vollständig durch das Reservoir eingekapselt ist, und
ein Band, mit dem die äußere Fläche des Reservoirs und des Antriebs ausgestattet sind, das den Antrieb mit dem Reservoir verbindet;
wobei die Darreichungsform so gestaltet ist, dass sie die Formulierung mit aktivem Mittel aus dem Inneren des Reservoirs in einer kontrollierten Menge ausstößt, nachdem die Darreichungsform dem Wirkungsort zugeführt wurde.

2. Darreichungsform nach Anspruch 1, wobei das Band ein gedrucktes Band, ein extrusionsbeschichtetes Band, :ein beschichtetes Band, ein siebdruckbeschichtetes Band, ein bürstenstrichbeschichtetes Band, ein bemaltes Band oder ein angestrichenes Band ist.

3. Darreichungsform nach Anspruch 1, wobei das Band ein Band umfasst, der kontinuierlich um die Darreichungsform in dem Bereich gebildet ist, wo der Antrieb und das Reservoir zusammenkommen.

4. Darreichungsform nach Anspruch 1, wobei das Band ein Material umfasst, das ausgewählt wird aus der Gruppe bestehend aus Polyethylen, Polystyrol, Ethylen-Vinyl-Acetat-Copolymer, Polycaprolacton: und auf Polyester basierende Elastomere, Polysaccharide, Cellulosederivate, pulverförmige Cellulose, mikrokristalline Cellulose, Celluloseacetat, Celluloseacetatpseudolatex, Celluloseacetatpropionat, Celluloseacetatbutyrat, Ethylcellulose, Ethylcellulosspseudolatex, Nitrocellulose, Polymilchsäure, Polyglykolsäure, Polymilchsäure-Glykolsäure-Copolymere, Kollagen, Polycaprolacton, Polyvinylalkohol, Polyvinylacetat, Polyethylenvinylacetat, Polyethylenterephthalat, Polybutadienstyrol, Polyisobutylen, Polyisobutylen-Isopren-Copolymer, Polyvinylchlorid, Polyvinylidenchlorid-Vinylchlorid-Copolymer, Copolymere aus Acrylsäure- und Methacrylsäureester, Copolymere aus :Methylmethacrylat und Ethylacrylat, Latex von Acrylatestern, Polypropylen, Copolymere aus Propylenoxid und Ethylenoxid, Propylenoxidethylenoxidblockcopolymere, Ethylenvinylalkoholcopolymer, Polysulfon, Ethylenvinylalkoholcopolymer, Polyxylylen, Polyamide, natürliche und synthetische Wachse, Karnaubawachs, Petroleumwachs, weißer oder gelber Bienenwachs, Kastorwachs, Kandelillawachs, Reiskleiewachs, mikrokristallines Wachs, Stearylalkohol, Cetylalkohol, gebleichter Schellack, veresterter Schellack, Chitin, Chitosan, Siliciumdioxid, Polyalkoxysilane, Poyldimethylsiloxan, Polyethylenglykol-Silikonelastomere, quervernetzte Gelatine, Zein, elektromagnetisch bestrahlte quervernetzte Acryle, Silikone, oder Polyester, thermisch vernetzte Acryle, Sllikone oder Polyester, Butadien-Styrol-Kautschuk, Glycerinester von teilweise dimerisiertes Kolphonium, Glycerinester von teilweise hydriertem Holzkolophonium, Glycerinester von Kiefemölkolophonium, Pentaerythritolester von teilweise hydriertem Holzkolophonium, Pentaerythritolester von Holzkolophonium, natürliches oder synthetisches Terpenharz, und Mischungen der oben genannten Materialien.

5. Darreichungsform nach Anspruch 1, wobei das Band ein Einfaßband umfasst.

6. Darreichungsform nach Anspruch 1, wobei das Band ein vorgeformtes Band umfasst.

7. Darreichungsform nach Anspruch 1, wobei der osmotische Antrieb eine dehnbare osmotische Zusammensetzung umfasst.

8. Darreichungsform nach Anspruch 1, wobei der osmotische Antrieb eine Barriereschicht oder eine äußere Beschichtung umfasst, die die Migration einer Formulierung mit aktivem Mittel aus dem Reservoir in den osmotischen Antrieb begrenzt.

9. Darreichungsform nach Anspruch 1, wobei das Reservoir ein wasserdurchlässiges Material umfasst.

10. Darreichungsform nach Anspruch 1, wobei das Reservoir ein Material umfasst, das im wesentlichen gegenüber Wasser undurchlässig ist.

11. Darreichungsform umfassend
ein Reservoir, das die Formulierung mit aktivem Mittel:enthält,
einen osmotischen Antrieb, der teilweise innerhalb einer Öffnung positioniert ist, die innerhalb des Reservoirs gebildet ist und der osmotische Antrieb nicht vollständig durch das Reservoir eingekapselt ist,
ein Band, mit dem die äußere Fläche des Reservoirs und des Antriebs ausgestattet sind, der den Antrieb mit dem Reservoir verbindet;
eine Membran zur Mengenkontrolle, und
eine Auslassöffnung durch die die Formulierung mit aktivem Mittel freigesetzt werden kann.

12. Verfahren zur Herstellung einer Darreichungsform, die die kontrollierte Freisetzung einer Formulierung mit aktivem Mittel bereitstellt, umfassend:
Bereitstellen eines Reservoirs mit einer Öffnung, die eine Größe aufweist und so geformt ist, um einen osmotischen Antrieb aufzunehmen, Bereitstellen :eines osmotischen Antriebs,
Positionieren des Antriebs innerhalb der Öffnung des Reservoirs, so dass der Antrieb teilweise innerhalb des:Reservoirs positioniert ist und der Antrieb nicht vollständig durch das Reservoir eingekapselt ist, und
Verbinden des Antriebs mit dem Reservoir.

13. Verfahren nach Anspruch 12, wobei das Verbinden des Antriebs mit dem Reservoir umfasst, Bedrucken des Bandes, Extrusionsbeschichten des Bandes, Beschichten des Bandes, Siebdruckbeschichten des Bandes, Bürstenstrichbeschichten des Bandes, Besprühen des Bandes und Bemalen des Bandes.

14. Verfahren nach Anspruch 12, wobei das Verbinden des Antriebs mit dem Reservoir das Formen des Bandes kontinuierlich um die Darreichungsform in dem Bereich umfasst, wo der Antrieb und das Reservoir zusammenkommen.

15. Verfahren nach Anspruch 12, wobei das Band ein Material umfasst, das ausgewählt wird aus der Gruppe bestehend aus Polyethylen, Polystyrol, Ethylen-Vinyl-Acetat-Copolymer, Polycaprolacton und auf Polyester basierende: Elastomere, Polysaccharide, Cellulosederivate, pulverförmige Cellulose, mikrokristalline Cellulose, Celluloseacetat, Celluloseacetatpseudolatex, Celluloseacetatpropionat, Celluloseacetatbutyrat, Ethylcellulose, Ethylcellulosspseudolatex, Nitrocellulose, Polymilchsäure, Polyglykolsäure, Polymilchsäure-Glykolsäure-Copolymere, Kollagen, Polycaprolacton, Polyvinylalkohol, Polyvinylacetat, Polyethylenvinylacetat, Polyethylenterephthalat, Polybutadienstyrol, Polyisobutylen, Polyisobutylen-Isopren-Copolymer, Polyvinylchlorid, Polyvinylidenchlorid-Vinylchlorid-Copolymer, Copolymere aus Acrylsäure- und Methacrylsäureester, Copolymere aus Methylmethacrylat und Ethylacrylat, Latex von Acrylatestern, Polypropylen, Copolymere aus Propylenoxid und Ethylenoxid, Propylenoxidethylenoxidblockcopolymere, Ethylenvinylalkoholcopolymer, Polysulfon, Ethylenvinylalkoholcopolymer, Polyxylylen, Polyamide, natürliche und synthetische Wachse, Karnaubawachs, Petroleumwachs, weißer oder gelber Bienenwachs, Kastorwachs, Kandelillawachs, Reiskleiewachs, mikrokristallines Wachs, Stearylalkohol, Cetylalkohol, gebleichter Schellack, veresterter Schellack, Chitin, Chitosan, Siliciumdioxid, Polyalkoxysilane; Poyldimethylsiloxan, Polyethylenglykol-Silikonelastomere, quervernetzte Gelatine; Zein, elektromagnetisch bestrahlte quervernetzte Acryle, Silikone, oder Polyester, thermisch vernetzte Acryle, Silikone oder Polyester, Butadien-Styrol-Kautschuk, Glycerinester von teilweise dimerisiertes Kolphonium, Glycerinester von teilweise hydriertem Holzkolophonium, Glycerinester von Kiefemölkolophonium, Pentaerythritolester von teilweise hydriertem Holzkolophonium, Pentaerythritolester von Holzkolophonium, natürliches oder synthetisches Terpenharz, und Mischungen der oben genannten Materialien.

16. Verfahren nach Anspruch 12, wobei das Band ein Einfaßband umfasst.

17. Verfahren nach Anspruch 12, wobei das Band ein vorgeformtes Band umfasst.

18. Verfahren nach Anspruch 12, außerdem umfassend das Einfüllen der Formulierung mit aktivem Mittel in das Reservoir.

19. Verfahren nach Anspruch 12, außerdem umfassend
Konfigurieren der Darreichungsform, so dass eine Auslassöffnung in dem Reservoir eingeschlossen oder gebildet wird, um die Freisetzung der Formulierung mit aktivem Mittel zu ermöglichen.

20. Verfahren nach Anspruch 12, wobei ein Antrieb bereitgestellt wird, umfassend
Bereitstellen eines osmotischen Antriebs, der eine Membran zur Mengenkontrolle umfasst.

21. Verfahren nach Anspruch 20, wobei die Membran zur Mengenkontrolle über wenigstens einen Teil des osmotischen Antriebs gebildet oder positioniert ist, der nicht durch das Reservoir eingekapselt ist.

22. Verfahren nach Anspruch 20, wobei die Membran zur Mengenkontrolle sowohl über einen exponierten Teil des osmotischen Antriebs als auch dem Reservoir gebildet oder positioniert ist.

23. Verfahren nach Anspruch 20, wobei der osmotische Antrieb außerdem eine Barriereschicht umfasst.

24. Verfahren nach Anspruch :23 außerdem umfassend:
Ausrichten des osmotischen Antriebs bevor er innerhalb des Reservoirs positioniert wird, so dass nachdem der Antrieb innerhalb der Öffnung des Reservoirs positioniert ist, die Barriereschicht der Formulierung mit aktivem Mittel gegenüberliegt.

25. Verfahren nach Anspruch 23, wobei die Barriereschicht eine Barriereschicht umfasst, die gegenüber der Permeation der Formulierung mit aktivem Mittel beständig ist.

## Revendications

1. - Forme posologique configurée pour assurer la libération contrôlée d'une formulation d'agent actif, comprenant :
- un réservoir contenant une formulation d'agent actif ;
- un moteur osmotique partiellement positionné à l'intérieur du réservoir, et le moteur n'étant pas complètement encapsulé par le réservoir ; et
- une bande disposée sur une surface externe du réservoir et du moteur qui lie le moteur au réservoir ;
la forme posologique étant configurée pour expulser la formulation d'agent actif à partir de l'intérieur du réservoir à une vitesse contrôlée après l'administration de la forme posologique à un environnement d'opération.

2. - Forme posologique selon la revendication 1, dans laquelle la bande est une bande imprimée, une bande revêtue par extrusion, une bande revêtue par sérigraphie, une bande revêtue à la brosse, une bande revêtue par pulvérisation ou une bande peinte.

3. - Forme posologique selon la revendication 1, dans laquelle la bande comprend une bande formée de façon continue autour de la forme posologique dans une zone où le moteur et le réservoir se réunissent.

4. - Forme posologique selon la revendication 1, dans laquelle la bande comprend une matière choisie dans le groupe constitué par le polyéthylène, le polystyrène, les copolymères éthylène-acétate de vinyle, les élastomères à base de polycaprolactone et de polyester, les polysaccharides, les produits cellulosiques, la cellulose pulvérulente, la cellulose microcristalline, l'acétate de cellulose, le pseudolatex d'acétate de cellulose, l'acétate propionate de cellulose, l'acétate butyrate de cellulose, l'éthylcellulose, le pseudolatex d'éthyl cellulose, la nitrocellulose, l'acide polylactique, l'acide polyglycolique, les copolymères polylactide glycolide, le collagène, la polycaprolactone, l'alcool polyvinylique, le poly(acétate de vinyle), le polyéthylène acétate, le vinyle téréphtalate, le polybutadiène styrène, le polyisobutylène, le copolymère polyisobutylène isoprène, le poly(chlorure de vinyle), le copolymère chlorure de polyvinylidène-chlorure de vinyle, les copolymères d'esters d'acide acrylique et d'acide méthacrylique, les copolymères de méthacrylate de méthyle et d'acrylate d'éthyle, le latex d'esters acrylates, le polypropylène, les copolymères d'oxyde de propylène et d'oxyde d'éthylène, les copolymères à blocs oxyde de propylène-oxyde d'éthylène, le copolymère éthylène alcool vinylique, la polysulfone, le copolymère éthylène alcool vinylique, les polyxylylènes, les polyamides, les cires naturelles et synthétiques, la paraffine, la cire de carnauba, la cire de pétrole, la cire d'abeilles blanche ou jaune, la cire de ricin, la cire de candelilla, la cire de son de riz, la cire microcristalline, l'alcool stéarylique, l'alcool cétylique, la gomme laque blanchie, la gomme laque estérifiée, la chitine, le chitosan, les silices, les polyalcoxysilanes, le polydiméthyl siloxane, les élastomères polyéthylène glycol-silicone, la gélatine réticulée, la zéine, les acryliques, silicones ou polyesters réticulés par rayonnement électromagnétique, les acryliques, silicones ou polyesters réticulés thermiquement, le caoutchouc butadiène-styrène, l'ester de glycérol de la colophane partiellement dimérisée, l'ester de glycérol de la colophane de bois partiellement hydrogénée, l'ester de glycérol de la colophane de tallol, l'ester de glycérol de la colophane de bois, l'ester de pentaérythritol de la colophane de bois partiellement hydrogénée, l'ester de pentaérythritol de la colophane de bois, la résine terpénique naturelle ou synthétique et des mélanges de ces matières.

5. - Forme posologique selon la revendication 1, dans laquelle la bande comprend un ruban.

6. - Forme posologique selon la revendication 1, dans laquelle la bande comprend une bande préformée.

7. - Forme posologique selon la revendication 1, dans laquelle le moteur osmotique comprend une composition osmotique expansible.

8. - Forme posologique selon la revendication 1, dans laquelle le moteur osmotique comprend une couche barrière ou un revêtement externe qui limite la migration d'une formulation d'agent actif à partir du réservoir dans le moteur osmotique.

9. - Forme posologique selon la revendication 1, dans laquelle le réservoir comprend une matière perméable à l'eau.

10. - Forme posologique selon la revendication 1, dans laquelle le réservoir comprend une matière qui est sensiblement imperméable à l'eau.

11. - Forme posologique comprenant :
- un réservoir contenant une formulation d'agent actif ;
- un moteur osmotique partiellement positionné à l'intérieur d'une ouverture formée à l'intérieur du réservoir, et le moteur osmotique n'étant pas complètement encapsulé par le réservoir ;
- une bande disposée sur une surface externe du réservoir et du moteur qui lie le moteur au réservoir ;
- une membrane contrôlant la vitesse ; et
- un orifice de sortie à travers lequel la formulation d'agent actif peut être administrée.

12. - Procédé de fabrication d'une forme posologique assurant la libération contrôlée d'une formulation d'agent actif, comprenant les opérations consistant à :
- se procurer un réservoir ayant une ouverture qui est dimensionnée et formée pour recevoir un moteur osmotique ;
- se procurer un moteur osmotique ;
- positionner le moteur à l'intérieur de l'ouverture du réservoir de telle sorte que le moteur est partiellement postionné à l'intérieur du réservoir, et le moteur n'étant pas complètement encapsulé par le réservoir ; et
- lier le moteur au réservoir par une bande.

13. - Procédé selon la revendication 12, dans lequel l'opération consistant à lier le moteur au réservoir par une bande comprend l'impression de la bande, le revêtement par extrusion de la bande, le revêtement par sérigraphie de la bande, le revêtement à la brosse de la bande, le revêtement par pulvérisation de la bande ou la peinture de la bande.

14. - Procédé selon la revendication 12, dans lequel l'opération consistant à lier le moteur au réservoir par une bande comprend la formation d'une bande de façon continue autour de la forme posologique dans une zone où le moteur et le réservoir se réunissent.

15. - Procédé selon la revendication 12, dans lequel la bande comprend une matière choisie dans le groupe constitué par le polyéthylène, le polystyrène, les copolymères éthylène-acétate de vinyle, les élastomères à base de polycaprolactone et de polyester, les polysaccharides, les produits cellulosiques, la cellulose pulvérulente, la cellulose microcristalline, l'acétate de cellulose, le pseudolatex d'acétate de cellulose, l'acétate propionate de cellulose, l'acétate butyrate de cellulose, l'éthyl cellulose, le pseudolatex d'éthyl cellulose, la nitrocellulose, l'acide polylactique, l'acide polyglycolique, les copolymères polylactide glycolide, le collagène, la polycaprolactone, l'alcool polyvinylique, le poly(acétate de vinyle), le polyéthylène acétate de vinyle, le polyéthylène téréphtalate, le polybutadiène styrène, le polyisobutylène, le copolymère polyisobutylène isoprène, le poly(chlorure de vinyle), le copolymère polychlorure de vinylidène-chlorure de vinyle, les copolymères d'esters d'acide acrylique et d'acide méthacrylique, les copolymères de méthacrylate de méthyle et d'acrylate d'éthyle, le latex d'esters acrylates, le polypropylène, les copolymères d'oxyde de propylène et d'oxyde d'éthylène, les copolymères à blocs oxyde de propylène oxyde d'éthylène, le copolymère éthylène alcool vinylique, la polysulfone, le copolymère alcool vinylique, les polyxylylènes, les polyamides, les cires naturelles et synthétiques, la paraffine, la cire de carnauba, la cire de pétrole, la cire d'abeilles blanche ou jaune, la cire de ricin, la cire de candelilla, la cire de son de riz, la cire microcristalline, l'alcool stéarylique, l'alcool cétylique, la gomme laque blanchie, la gomme laque estérifiée, la chitine, le chitosan, les silices, les polyalcoxysilanes, le polydiméthyl siloxane, les élastomères polyéthylène glycol-silicone, la gélatine réticulée, la zéine, les acryliques, silicones ou polyesters réticulés par rayonnement électromagnétique, les acryliques, silicones ou polyesters réticulés thermiquement, le caoutchouc butadiène-styrène, l'ester de glycérol de là colophane partiellement dimérisée, l'ester de glycérol de la colophane de bois partiellement hydrogénée, l'ester de glycérol de la colophane de tallol, l'ester de glycérol de la colophane de bois, l'ester de pentaérythritol de la colophane de bois partiellement hydrogénée, l'ester de pentaérythritol de la colophane de bois, la résine terpénique naturelle ou synthétique et des mélanges de ces matières.

16. - Procédé selon la revendication 12, dans lequel la bande comprend un ruban.

17. - Procédé selon la revendication 12, dans lequel la bande comprend une bande préformée.

18. - Procédé selon la revendication 12, comprenant en outre la charge d'une formulation d'agent actif dans le réservoir.

19. - Procédé selon la revendication 12, comprenant en outre la configuration de la forme posologique de telle sorte qu'un orifice de sortie est inclus ou formé dans le réservoir pour permettre l'administration de la formulation d'agent actif.

20. - Procédé selon la revendication 12, dans lequel l'opération consistant à se procurer un moteur comprend l'opération consistant à se procurer un moteur osmotique qui comprend une membrane contrôlant la vitesse.

21. - Procédé selon la revendication 20, dans lequel la membrane contrôlant la vitesse est formée ou positionnée sur au moins une partie du moteur osmotique qui n'est pas encapsulée par le réservoir.

22. - Procédé selon la revendication 20, dans lequel la membrane de contrôle est formée ou positionnée sur à la fois une partie exposée du moteur osmotique et le réservoir.

23. - Procédé selon la revendication 20, dans lequel le moteur osmotique comprend en outre une couche barrière.

24. - Procédé selon la revendication 23, comprenant en outre :
l'orientation du moteur osmotique avant qu'il ne soit positionné à l'intérieur du réservoir de telle sorte qu'après que le moteur soit positionné à l'intérieur de l'ouverture du réservoir, la couche barrière fait face à la formulation d'agent actif.

25. - Procédé selon la revendication 23, dans lequel la couche barrière comprend une couche barrière qui est résistante à la perméation par la formulation d'agent actif.
